(19) Europäisches Patentamt
European Patent Office
Office européen des brevets

(11) **EP 1 323 459 A1**

(12) **EUROPEAN PATENT APPLICATION**
published in accordance with Art. 158(3) EPC

(43) Date of publication:
**02.07.2003 Bulletin 2003/27**

(51) Int Cl.⁷: **B01D 39/14**, B01D 39/16,
A61L 9/01, A61L 9/16

(21) Application number: **01947907.0**

(22) Date of filing: **06.07.2001**

(86) International application number:
**PCT/JP01/05916**

(87) International publication number:
**WO 02/005927 (24.01.2002 Gazette 2002/04)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **13.07.2000 JP 2000212672**
**14.08.2000 JP 2000245553**

(71) Applicant: **BRIDGESTONE CORPORATION**
**Tokyo 104-0031 (JP)**

(72) Inventors:
• **MORI, Hisashi**
**Sakae-ku, Yokohama-shi, Kanagawa 247-000 (JP)**
• **NEMOTO, Yasushi**
**Fujisawa-shi, Kanagawa 251-0042 (JP)**
• **KUWABARA, Tadashi**
**Yokosuka-shi, Kanagawa 238-0004 (JP)**

(74) Representative: **ter Meer, Nicolaus**
**Ter Meer Steinmeister & Partner GbR,**
**Mauerkircherstrasse 45**
**81679 München (DE)**

(54) **DEODORIZING FILTER MATERIAL**

(57)     A deodorizing filter material characterized by comprising a pair of two-dimensional reticular skeletons disposed parallel and spaced a predetermined distance apart from each other, a limitless number of connecting threads which connect the pair of two-dimensional reticular skeletons to form a three-dimensional fiber skeleton, to which a deodorant is adhered, the deodorizing filter material having excellent deodorizing performance superior to one using a polyurethane form matrix, without producing problems such as those concerning construction, production, and strength, and by reason of the same laminar flow effect as in the case of using a matrix of honeycomb construction. Further, a renewable deodorizing filter material characterized in that a deodorant having a gas adsorbing action due to ion exchange function is adhered to the skeleton surface of a filter matrix of three-dimensional construction makes it possible to recycle filters whose deodorizing performance has degraded.

**FIG.1**

EP 1 323 459 A1

**Description**

TECHNICAL FIELD

This invention concerns a deodorizing filter material suitably used, for example, in filters removing trace amount of gas ingredients for use in clean rooms, air cleaners, air conditioners, filters for ventilation and air conditioning, air cleaning filters upon introduction of external air into vehicle cabins (cabin filters) and, further, filters for removing impurities in external air upon supply of oxygen for fuel cells.

BACKGROUND ART

Various deodorizing filters such as filters of removing trace amount of gas ingredients for use in clean rooms, air cleaners, air conditioners, filters for ventilation and air conditioning and air cleaning filters upon introduction of external air into cabins (cabin filters) have been used so far, and filters for removing impurities in external air are required also for fuel cells noted in recent years upon supply of oxygen to the cells, and the application use for the deodorizing filters has been generalized more and more. The deodorizing filters mentioned herein include not only those removing odor ingredients but also include those collecting and removing gas ingredients other than odor ingredients.

Heretofore, various kinds of filter materials constituting such deodorizing filters have been proposed and put to practical use depending on the application uses thereof and, generally, any of them has a deodorant deposited to air permeable matrixes.

In this case, for the air permeable matrixes, those of a honeycomb structure using paper or aluminum foil as a base material, various kinds of resin nets and polyurethane foams having three-dimensional reticular skeletons are used and deodorizing filter materials are constituted by depositing deodorants to the skeletons of the air permeable matrixes.

However, while a filter using a matrix of a honeycomb structure comprising paper or aluminum foil as a base material has a merit that the pressure loss is low due to the laminar effect of the honeycomb structure, it is necessary to previously be corrugated by coating a deodorant to a flat-shape base material, and then be laminated and cut cross sectionally upon manufacture and, accordingly, it involves a problem that the deodorant can not be deposited sufficiently. Further, it also involves problems that the honeycomb meshes tend to be crushed and interlayer peeling occurs in the laminating direction.

Further, after fabricating the base material into a honeycomb structure, it is also possible to deposit a deodorant, for example, by impregnating the honeycomb structure with a slurry of a deodorant, or by using a binder. However, this results in a problem upon dipping treatment which dipping the slurry kneaded a deodorant and a binder ingredient or the binder to the honeycomb structure. In this case, the honeycomb is crushed and the shape is not restored or the productivity is poor since it relies on batchwise production because the roll impregnation method can not be adopted.

Further, for the filters of the honeycomb structure, while those molded into a honeycomb shape by extrusion molding of a slurry formed by kneading an adsorbent (deodorant) and a binder ingredient has been proposed, since the material of the filter has a brittle property, the filter involves a drawback that it is sensitive to impact shocks and remarkably poor in handlability.

On the other hand, in the case of using polyurethane foams having a three-dimensional reticular skeleton, it involve a problem that since a foamed body is obtained by forming cells generally by chemical blowing, the cell control is difficult, and pressure loss to the honeycomb structure is increased because no laminar effect as in the honeycomb structure can be obtained.

Accordingly, it has been demanded for the development of deodorizing filter materials, without causing a problem in view of the structure such as interlayer peeling, a problem in view of the productivity and, further, a problem in view of strength and having excellent deodorizing performance superior to those using polyurethane foam matrixes due to the laminar flow effect comparable with the case of using the matrix of the honeycomb structure.

On the other hand, coconut shell activated carbon has generally been used as the deodorant deposited to the air permeable matrix, and the coconut shell activated carbon has excellent gas adsorptivity and can provide high deodorizing performance.

However, such deodorizing filters, not being restricted to the coconut shell activated carbon, deteriorate the deodorizing performance with lapse of time along with gas adsorption and the deodorizing performance thereof is lowered to such an extent as incapable of attaining an intended purpose in a relatively short time depending on application uses. Accordingly, the filter materials have to be exchanged frequently for maintaining a sufficient deodorizing performance, which is extremely uneconomical.

In view of the above, while it has been demanded to regenerate the deodorizing filter of degraded deodorizing performance and use the same again for air cleaning or the like, the existent deodorizing filter materials using the coconut shell activated carbon or the like involve a problem that regeneration is scarcely possibly or need complicate operation or large scale operation for regeneration. So, the recyclic use of the filter material is not practical at present.

Accordingly, it has been demanded for a deodorizing filter material capable of regenerating favorable deodorizing performance by a relatively simple and convenient method and capable of recyclic use has been demanded.

DISCLOSURE OF INVENTION

This invention has been accomplished in view of the foregoing situations and it is a first object thereof to provide a deodorizing filter without causing a problem in view of the structure such as interlayer peeling, a problem in view of the productivity and, further, a problem in view of strength and having excellent deodorizing performance superior to those using polyurethane foam matrixes due to the laminar flow effect comparable with the case of using the polyurethane foam matrix.

Further, it is a second object of the invention to provide, in view of the foregoing situations, a deodorizing filter material capable of favorably regenerating the deodorizing performance by a relatively simple and convenient method and capable of recyclic use.

For attaining the foregoing first object, the present invention provides a deodorizing filter material as a first invention, in which a pair of two-dimensional reticular skeletons are disposed in parallel and spaced apart by a predetermined distance from each other, and the pair of two-dimensional reticular skeletons are connected to each other by a number of connecting threads to form a three-dimensional fiber skeletons, to which a deodorant is deposited

In the deodorizing filter material according to the first invention, a three-dimensional fiber skeleton in which a pair of two-dimensional reticular skeletons are disposed in parallel spaced apart by a predetermined distance from each other are connected by a number of connecting threads is used as the matrix to which the deodorant is deposited. Accordingly, a slurry of the deodorant or the binder can be applied easily to the matrix, for example, by a dipping treatment, and a filter of a honeycomb structure excellent in the productivity and the strength can be obtained at a high productivity by continuous production, without causing the problem in view of the structure such as interlayer peeling or crushing upon cutting, as well as excellent deodorizing performance can be provided superior to a case of using the polyurethane foam matrix by favorable laminar effect. In this case, in the deodorizing filter material of the first invention, since the slurry of the adsorbent can be impregnated between the fibers of the three-dimensional fibers skeleton matrix and more deodorant can be deposited, the deodorizing performance can be improved more than in a case of using the existent honeycomb structure.

Further, the present inventors have made an earnest study for attaining the second object and, as a result, have accomplished the following second invention based on the finding that sufficient deodorizing performance can be recovered and the filter can be regenerated easily by using a deodorant having a gas adsorbing effect by ion exchanging function as the deodorant, and constituting a deodorizing filter material by depositing the deodorant on the surface of the skeleton of a filter matrix having a three-dimensional structure and by way of a simple and convenient method, for example, by treating with water or an aqueous weakly basic or weakly acidic property solution.

That is, the present invention provides as the second invention for attaining the second object a regenerative deodorizing filter material in which a deodorant having a gas adsorbing effect due to ion exchange function is deposited to the surface of a skeleton of a filter matrix having a three-dimensional structure.

BRIEF DESCRIPTION OF DRAWINGS

FIG. 1 is a fragmentary enlarged view showing an example of a three-dimensional fiber skeleton constituting a deodorizing filter material according a first invention.

FIG. 2 is a schematic view showing a testing equipment used for a pressure loss test in Example 1 and Comparative Example 1.

FIG. 3 is a schematic view showing a testing apparatus used for a deodorizing performance test in Example 1 and Comparative Example 1.

FIG. 4 is a graph showing an ammonia removable ratio in an air cleaning test conducted in Example 2 and Comparative Example 2.

FIG. 5 is a graph showing an acetoaldehyde removable ratio in an air cleaning test conducted in Example 2 and Comparative Example 2.

FIG. 6 is a graph showing an acetic acid removable ratio in an air cleaning test conducted in Example 2 and Comparative Example 2.

BEST MODE FOR CARRYING OUT THE INVENTION

Hereinafter, the present invention is to be explained for the details of each of the first invention and the second invention.

[First Invention]

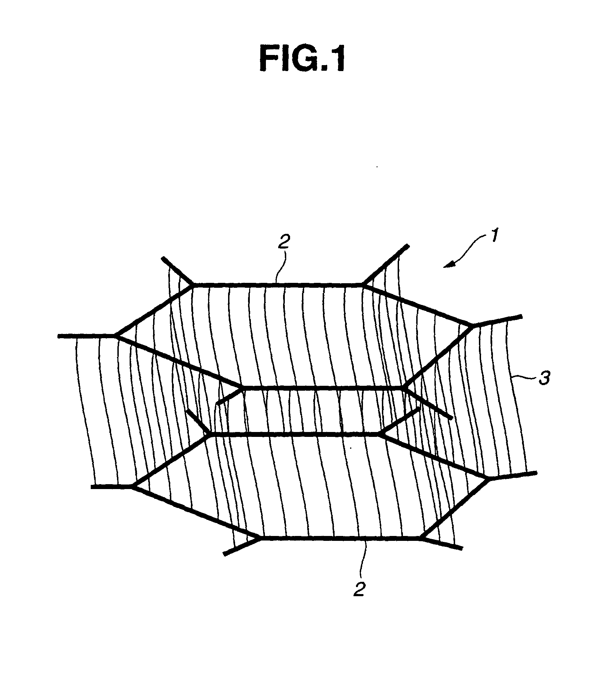

A deodorizing filter material according to the first invention uses a unique three-dimensional fiber skeleton as a matrix and a deodorant is deposited to the skeleton as described above. For example, as shown in FIG. 1, it comprises, as a matrix, a three-dimensional fiber skeleton 1 comprising a pair of two-dimensional reticular skeletons 2, 2 each having a hexagonal reticular shape disposed in parallel and spaced apart by a predetermined distance with each other and a number of connecting threads 3 connecting the pair of two-dimensional reticular skeletons 2, 2 is used as a matrix, and deodorant is deposited to the matrix.

The materials of the fibers forming three-dimensional fiber skeleton 1 have no particular restriction and are properly selected depending on the application use of the filter and, specifically, they include, for example, synthetic fibers such as polyester fibers, polyamide fibers, polypropylene fibers, polyethylene fibers and acrylic fibers and one or more of them can be used. Particularly, polyester fibers, polyamide fibers, polypropylene fibers or composite fibers using two or more of them are used suitably in view of the fabricability upon forming the three-dimensional fiber skeleton structure, fabricability of the deodorizing filter and the durability of the filter.

The two-dimensional reticular skeleton 2, 2 forming the three-dimensional fiber skeleton 1 may comprise one single fibers but plural fibers twisted to each other into a fiber of a predetermined diameter are used preferably, which can impregnate the deodorant slurry to be described later in the skeletons of the two-dimensional reticular skeletons 2, 2 and increase the deposition amount of the deodorant.

There is no particular restriction on the fiber diameter of the fibers constituting the two-dimensional reticular skeletons 2, 2 and usually it is preferably about from 0.001 to 0.5 mm and, particularly, 0.01 to 0.1 mm. In a case of forming the skeleton with a fiber formed by twisting a plurality of fibers, the skeleton with the fiber diameter described above can be formed by twisting about 10 to 100 fibers of 5 - 1000 denier, particularly, 100 - 500 denier to each other.

The two-dimensional reticular skeletons 2, 2 are formed in a reticular shape as shown in FIG. 1, and the lattice configuration of the two-dimensional reticular skeletons 2, 2 may be any of shapes such as trigonal, tetragonal, pentagonal, hexagonal or more polygonal shape and it is particularly preferred for a reticular structure of a hexagonal lattice configuration as shown in FIG. 1 and constitution of a honeycomb-like filter is preferred for example, in view of the shape stability upon fabrication and effective suppression of pressure loss when used as a filter.

The lattice size for the two-dimensional reticular skeletons 2, 2 can be set properly in accordance with the application use of the filter or the like with no particular restriction and, usually, it is preferably about from 1 to 15 mm and, particularly, from 4 to 7 mm. If the lattice diameter is less than 1 mm, pressure loss sometimes increases depending on the application use, whereas if it exceeds 15 mm, the contact efficiency between the gas ingredient and the adsorbent (deodorant) is sometimes lowered to deteriorate the gas removing performance.

Since each of the two-dimensional reticular skeletons 2, 2 forms the front surface or rear surface portion of the filter respectively, it is usually preferred that the fiber system, lattice configuration and lattice diameter for the two-dimensional reticular skeletons 2, 2 are substantially identical, that is in a substantially identical shape. Depending on the case, both of the two-dimensional reticular skeletons 2, 2 may be of shapes different from each other, and the deodorizing performance can be improved sometimes depending on the application use by making the shape of both of the two-dimensional reticular skeletons 2, 2 different from each other.

The two-dimensional reticular skeletons 2, 2 are disposed in parallel spaced apart by a predetermined distance from each other and connected by the connecting threads 3, in which the distance between the two-dimensional reticular skeletons 2, 2 constitute the thickness of the filter material. The distance between both of them two-dimensional reticular skeletons 2, 2, that is, the thickness of the filter material is properly selected depending on the application use and the like with no particular restriction and it is usually from 2 to 30 mm and, particularly, from 5 to 20 mm.

Then, the connecting threads 3 for connecting the two-dimensional reticular skeletons 2, 2 to each other may be formed of fibers of material different from that of the fibers constituting the two-dimensional particular skeletons 2, 2 but usually they are preferably formed of fibers comprising the identical material. Further, a number of connecting threads 3 are disposed between both of the two-dimensional reticular skeletons, they may be fibers independent from each other or fibers of one or plurality of fibers which are turned back to bridge both of the two-dimensional reticular skeletons 2, 2.

The fiber diameter for the connecting thread 3 is properly selected depending on the material or the like with no particular restriction and usually it is, preferably, from 5 to 1000 denier and, particularly form 100 to 500 denier. If the fiber diameter of the connecting thread is less than 5 denier, the connection strength between both of the two-dimensional reticular skeletons 2, 2 is insufficient to sometimes cause so-called "buckling" in the direction of thickness.

On the other hand, if it exceeds 1000 denier, it is difficult to reliably connect both of the two-dimensional reticular skeletons 2, 2 making it sometimes difficult to constitute the three-dimensional fiber skeleton 1.

In the deodorizing filter material according to the second invention, a deodorant is deposited to the three-dimensional fiber skeleton 1 formed by connecting the pair of two-dimensional reticular skeletons 2, 2 with the connecting threads.

There is no particular restriction on the deodorant, which can be selected properly depending on the application use of the filter or the like and can include, for example, various kinds of activated carbon such as coconut shell activated carbon, wooden activated carbon, spherical petroleum pitch activated carbon, and pellet molded activated carbon, inorganic synthetic chemical deodorant based on the material such as activated clay, zeolite, sepiolite, silica, alumina, $SiO_2$, ZnO and $TiO_2$, a $TiO_2$ series catalyst causing catalytic activity by photo-excited effect, metal catalyst carrying type deodorant formed by carrying noble metal or base metal catalyst such as Au, Ag, Ru, Rh, Pd, Pt, Mn, Ni, Co, alkali metals and alkaline earth metals on a porous body, a deodorant carrying, on a porous body, a compound of chemically decomposing and deodorizing the aimed gas ingredients by utilizing the chemical reaction such as neutralizing reaction or dehydrating condensation reaction, chelate resin, chelate compound bonded with metal ion by coordination bond, cation exchange resins having sulfonic acid groups or carboxylic acid groups to ion exchange functional groups based on a styrenic resin or acrylic resin, anion-exchange resins having quaternary ammonium groups or primary to tertiary amino groups, zeolite having cationic-exchange function, cationic-exchange inorganic synthetic chemical adsorbents such as Zr, Sn, Sb, Ti or Zn series, anionic-exchange inorganic synthetic chemicals such as Bi, Zr or Pb series, or amorphous ion exchange inorganic synthetic chemical adsorbents such as Sb, Bi series or $SiO_2$, ZnO.

There is no particular restriction on the method of depositing the deodorant to the three-dimensional fiber skeleton 1 and any known method can be adopted and, for example, a method of kneading the deodorant described above with a binder ingredient to prepare a slurry, and dipping and impregnating the same to the three-dimensional fiber skeleton 1, a method of dipping and impregnating only the binder ingredient into the three-dimensional fiber skeleton 1 to previously form a binder layer and then depositing the deodorant described above thereon and, further, a method of impregnating a slurry formed by kneading the binder ingredient and the deodorant into the three-dimensional fiber skeleton 1 and further depositing and drying an identical or other deodorant to the slurry before drying.

The binder for fixing the deodorant to the three-dimensional fiber skeleton 1 can include, for example, colloidal silica type inorganic binder such as silica sol or water glass (concentrated aqueous solution of sodium silicate) and, in a case where the working temperature of the filter is at a relatively low temperature, binders having pressure sensitive adhesive property or bonding property such as acrylic system, urethanic system, SBR system, NBR system or chloroprene system can also be used.

In the deodorizing filter material according to the first invention, an air permeable film may be formed optionally to one side, both sides or in the inside thereof and the film can include, for example, with no particular restriction, fluoro resin, polyamide resin, polyimide resin, polyester resin, polystyrene resin, polyolefin resin, polycarbonate resin, polysulfone resin, acryl resin, cellulose resin, vinyl chloride resin, polyacetal resin, polyurethane resin and copolymers thereof, as well as derivatives thereof and one or more of them can be used. Further, among them, the fluoro resin can include, polytetrafluoroethylene, polyhexafluoropropylene, ponidifluoroethylene, polyvinylidene fluoride and polyvinyl fluoride and copolymers thereof, and one or more of them can be used.

In the deodorizing filter material according to the first invention, the deodorant is deposited to the three-dimensional fiber skeleton 1 and, while the filter material can provide a favorable deodorizing performance by the filter material alone, it may be used optionally by laminating with one or more of other filter materials. For example, a deodorizing filter layer based on polyurethane foams having the three-dimensional reticular skeleton structure may be laminated or a known dust collecting filter layer having dust collecting function may be laminated to the filter material according to the present invention to add a dust collecting function in addition to the deodorizing function.

The other filter layer to be laminated may be on the one side or both sides of the filter material according to the first invention and may be in a bonded state by using an adhesive or non-bonded state by using a clip- or frame-filter connection member. In this case, when it is laminated in the non-bonded state by using the filter connection member, the filter material can be separated easily into each of structures, which can contribute to the improvement of recycling property, for example, by regeneration.

[Second Invention]

Then, a deodorizing filter material according to the second invention is to be described.

In the deodorizing filter material according to the second invention, a deodorant is deposited on the surface of the skeleton of the filter matrix having the three-dimensional structure, and a deodorant having a gas adsorbing effect by ion exchange function is used as the deodorant therefor as described above.

The filter matrix used for the deodorizing filter material according to the second invention may be any three-dimensional structure having a number of air vent apertures or air permeable connection channels and they can include, for example, a honeycomb structure using paper or aluminum foil as a base material, various kinds of resin nets having a three-dimensional reticular structure, polyurethane foams having a three-dimensional reticular skeleton with no cell membranes, and fibers skeletons molded into a three-dimensional structure and, particularly, the polyurethane foam having the three-dimensional reticular structure or fiber skeletons molded into the three-dimensional structure are used preferably since the strength during regenerative treatment is favorable and good deodorizing performance can be obtained.

The fiber skeleton molded into the three-dimensional structure has no particular restriction and the three-dimensional skeleton of a structure in which a pair of two-dimensional reticular skeletons are disposed spaced apart by a predetermined distance from each other and the pair of two-dimensional particular skeletons are connected by a number of connecting threads used for the deodorizing filter material according to the first invention can be used preferably. By using the three-dimensional fiber skeleton as the filter matrix, a great amount of the deodorant can be deposited, a filter material of a honeycomb structure can be constituted, and favorable deodorizing performance can be obtained reliably by favorable laminar effect or the deodorant deposited in a great amount, as well as it has favorable strength, so that regenerative operation can be conducted effectively with no crushing or fracture of the matrix during regeneration.

As the three-dimensional fiber skeleton, the three-dimensional fiber skeleton in FIG. 1 explained for the first invention can be exemplified. In this case, the thickness of the three-dimensional fiber skeleton 1, the structure, the shape, the material, the fiber diameter or the like of the two-dimensional reticular skeletons 2, 2 and the connecting threads 3 constituting the three-dimensional fiber skeleton 1 can be made identical with those of the first invention.

Then, the polyurethane foam used as the matrix for the deodorizing filter material according to the second invention has a three-dimensional reticular skeleton with no cell membranes, and commercial products, for example, "HR-06 to HR-50" manufactured by Bridgestone Co. can be used.

The polyurethane foam has no particular restriction and usually the number of cells is preferably from 5 to 50 PPI (Pores Per Inch) and, particularly, 6 to 30 PPI. If the number of cells is less than 5 PPI, the efficiency of contact with the gas ingredient may sometimes lowers depending on the application use failing to provide a sufficient deodorizing performance, whereas if it exceeds 50 PPI, the pressure loss of the filter is sometimes excessively high, which provides a problem in view of actual use depending on the application use.

In the deodorizing filter material according to the second invention, the deodorant is deposited to the surface of the skeleton of the filter matrix having the three-dimensional fiber skeleton, polyurethane foam or like other three-dimensional structure, and a deodorant having a gas adsorbing effect by ion exchange function is used as the deodorant in this invention.

For the deodorant, any of those having the gas adsorbing effect by the ion exchange function may be used, which may be either inorganic or organic type, with the inorganic type being used suitably in view of the fabricability. Further, the ion exchange type of the adsorbent may be a cationic-exchange type or anionic-exchange type.

Specifically, the inorganic adsorbent can include, for example, a cationic-exchange type such as silicon dioxide series, zirconium series, titanium series, antimony series and tin series or anionic-exchange type deodorant such as zinc oxide series, aluminum series and bismuth series, and one or more of them may be used alone or in combination. Further, the organic adsorbent can include, for example, a cationic-exchange resin comprising a styrene series resin or acryl series resin as a matrix in which sulfonic acid groups or carboxylic acid groups are present as ionic exchange functional groups, and an anionic-exchange resin having quaternary ammonium groups or primary to tertiary amino groups. Further, organic adsorbents such as cationic or anionic-exchange fibers, chelate resins and chelate resin and chelate compound can also be used. Further, a composite adsorbent formed by combining one or more of the inorganic adsorbents and one or more of the organic adsorbents can also be used.

There is no particular restriction on the method of depositing the deodorant to the filter matrix such as the three-dimensional fiber skeleton or the polyurethane foam, and the deodorant can be deposited on the filter matrix by using the same binder and by the same method as the binder illustrated for the first invention.

The deodorizing filter material according to the second invention can be regenerated for the deodorizing performance to a high level by applying the regenerative treatment even after the deodorizing performance is lowered. In this case, the method of the regenerating treatment is properly selected in accordance with the kind of the deodorant used with no particular restriction. A method of treating using water or an aqueous weakly basic or weakly acidic solution is adopted preferably and, particularly, by using the inorganic cationic or anionic adsorbent described above for the deodorant, the deodorizing function can be regenerated at a high level by a simple and convenient method. Further, the regenerating efficiency can be improved also by applying supersonic waves upon treatment with water or an aqueous weakly basic or weakly acidic solution.

EXAMPLE

The present invention is to be described more specifically with reference to examples and comparative examples, but the invention is not restricted to the following examples.

Example 1

Fibers of an average thickness of 1 mm formed by twisting polyester fibers at 250 denier by the number of 48 were knitted into a reticular form to form a plurality of hexagonal lattices each with an average inner size of 5.5 mm in the direction of the major diameter and an average inner size of 3.2 mm in the direction of the minor diameter to constitute a pair of two-dimensional reticular skeletons. The pair of two-dimensional reticular skeletons were disposed in parallel being spaced apart by 5 mm, and both of the two-dimensional reticular skeletons were connected to each other by polyester mono-filaments each of 150 denier to obtain a filter matrix comprising the three-dimensional fiber skeleton having the same structure as that shown in FIG. 1.

On the other hand, after mixing and kneading coconut shell activated products with an average grain size of 300 mesh with an acrylic binder "AE-932" (solid content 53%) manufactured by E-Tech Co. at a solid content ratio of 1 : 3.5, and water was added so as to provide 45% solid content to prepare a deodorant slurry. The matrix comprising the three-dimensional reticular skeleton was dipped in the slurry, the slurry was impregnated into the substrate and dried to deposit the coconut shell activated carbon, to obtain a deodorizing filter material according to the first invention having a honeycomb structure with the deposition amount of activated carbon of 500 g/m$^2$.

Comparative Example 1

For polyurethane foams having a three-dimensional reticular skeleton ["HR-08" manufactured by Bridgestone Co.] (8 mm thickness product), a deodorant slurry identical with that in the example was used and a coconut shell activated carbon was deposited in the same manner as in Example 1, to obtain a deodorizing filter material with the deposition amount of activated carbon of 225 g/m$^2$.

For both of the deodorizing filter materials obtained in Example 1 and Comparative Example 1, the following test was conducted to evaluate the pressure loss and the benzene deodorizing performance. The result is shown in Table 1.

[Test Method]

Pressure Loss Test

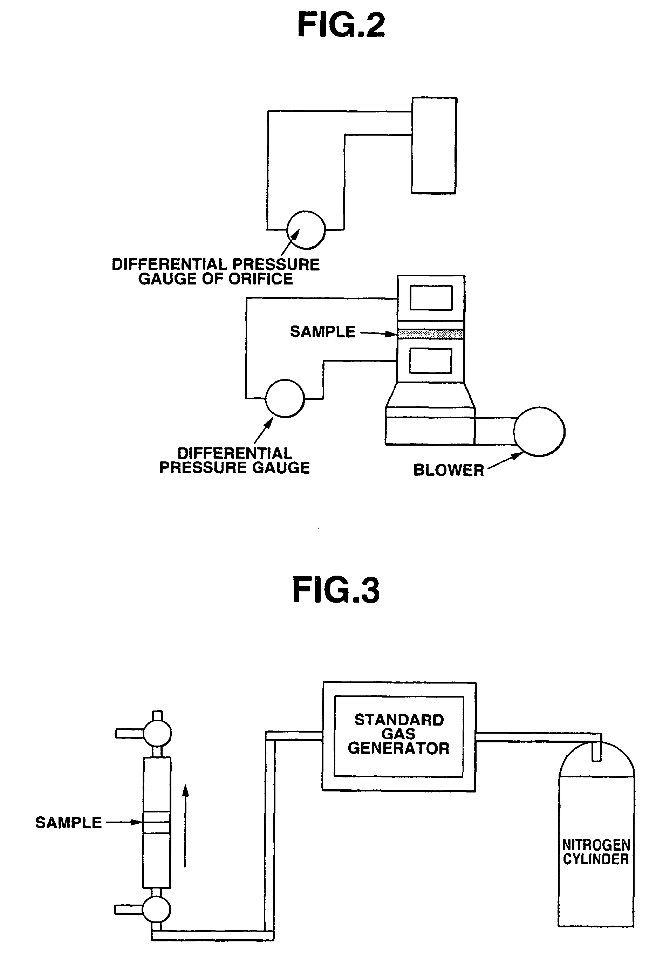

A testing equipment shown in FIG. 2 according to a test method specified by Air Cleaning Society was used and a test specimen (250 mm × 250 mm) was set as a sample to the testing equipment. In this state, a wind speed was set to 1 m/sec by controlling a blower while confirming a differential pressure gauge (window velocity gauge) of an orifice in this state, and the pressure loss is read by the differential pressure gauge.

Benzene Deodorizing Performance Test

The samples (5 mm thickness) manufactured in Example 1 and Comparative Example 1 were cut each to 40$\phi$, two sheets of the samples are stacked to each other, which is located about at the center of a glass column of 200 mm inner length and 40$\phi$ inner diameter shown in FIG. 3. A standard gas generation device manufactured by Gas Tech Co. is used to set the gas generation temperature to 35°C, a diffusion tube D-03 type was set, a nitrogen cylinder at a standard grade is used for a dilution gas and the dilution gas flow rate is set to 1 L/min.

As shown in FIG. 3, three way cocks are attached on both upper and lower ends of a glass column and a gas is vented toward the direction of the sample at the instance the inlet gas concentration reaches 30 ± 5 ppm. The benzene gas concentration at the exit of the sample and the benzene gas concentration at the inlet of the sample after lapse of 30 min are measured by a gas detection tube No. 121 manufactured by Gas Tech Co. to evaluate benzene removing ratio.

Table 1

| | Activated carbon deposition amount (g/m$^2$ dry) | Pressure loss (wind speed 1 m/sec hr) | Benzene removal ratio (%) |
|---|---|---|---|
| Example | 500 | 0.25 mmAq | 100 |
| Comparative Example | 225 | 0.30 mmAq | 55 |

As shown in Table 1, it was confirmed that since the deodorizing filter of the example according to the invention showed low pressure loss due to the laminar effect by the honeycomb structure and could deposit a great amount of activated carbon, it had excellent deodorizing performance.

Example 2

After mixing and kneading an SiO$_2$/ZnO system synthetic chemical deodorant (average grain size: 60 mesh) having ion exchange function with an acrylic binder "AE-932" (solid content 53%) manufactured by E-Tech Co. at a solid content ratio of 1 : 3.5, water was added so as to provide 45% solid content to prepare a deodorant slurry. The filter matrix comprising the same three-dimensional fiber skeleton as in Example 1 was dipped in the slurry, the slurry was impregnated into the substrate and dried to deposit the inorganic series synthetic chemical deodorant, to obtain a deodorizing filter material according to the second invention having a honeycomb structure with the deposition amount of the deodorant of 500 g/m$^2$.

Comparative Example 2

For polyurethane foams having a three-dimensional reticular skeleton ["HR-08", manufactured by Bridgestone Co.] (3 mm thickness product), an acrylic binder of 50% solid content "EW-2500" (manufactured by Soken Chemical & Engineering Co., Ltd.) was previously impregnated by 30 g/L (day), to which coconut shell activated carbon having an average grain size of 60 mesh with deposition of 2% by weight of orthophosphate to activated carbon was deposited, to obtain a deodorizing filter material with the deposition amount of activated carbon of 300 g/m$^2$.

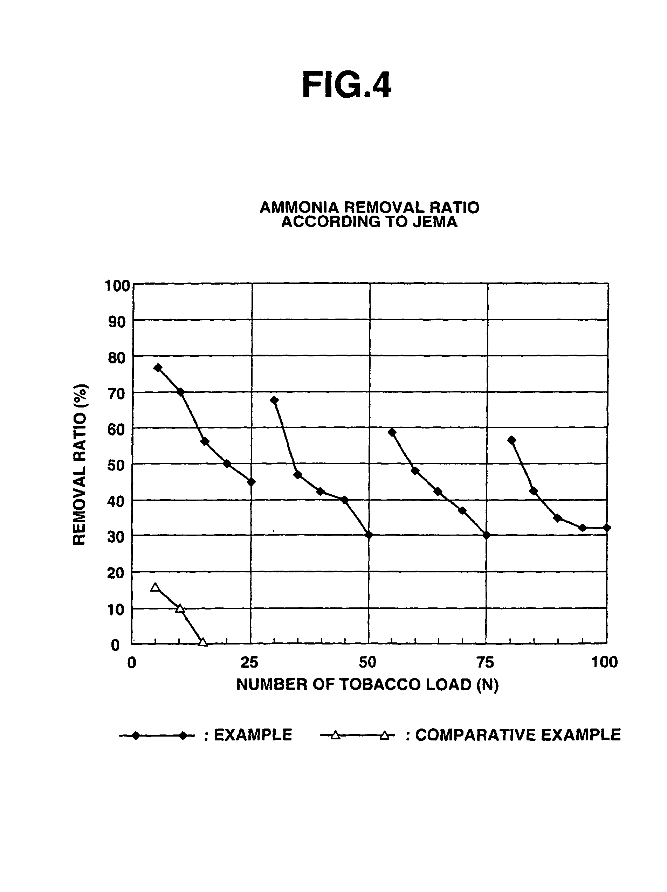

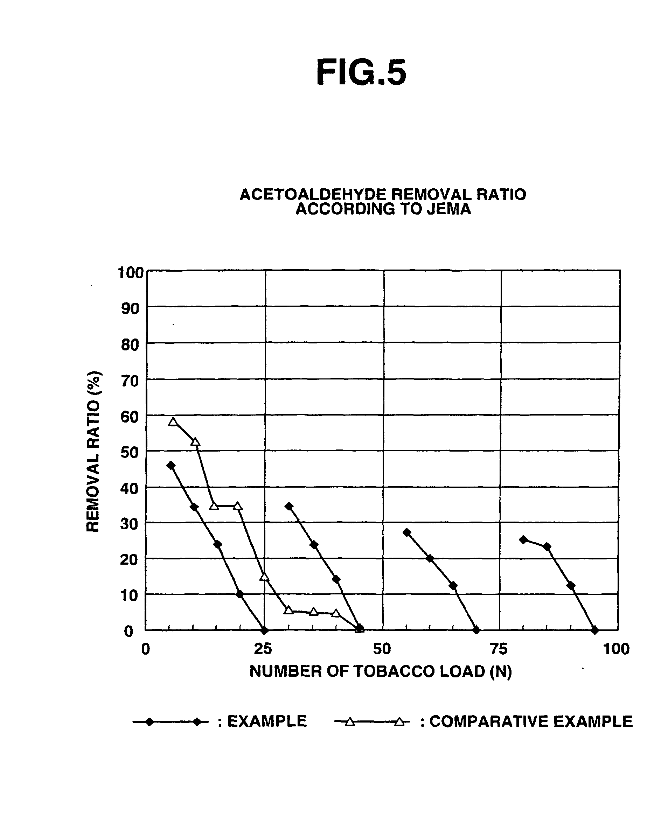

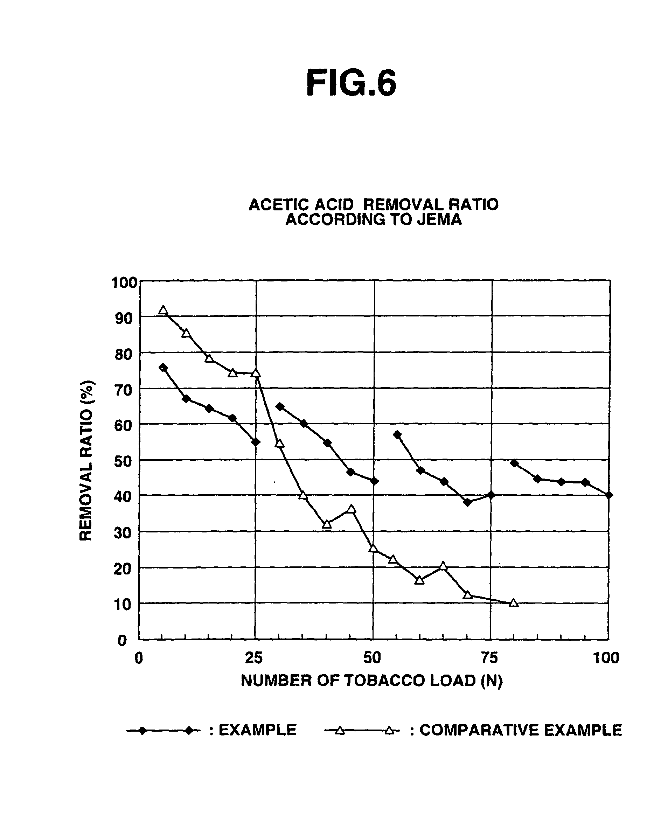

The deodorizing filter materials obtained in Example 2 and Comparative Example 2 were attached to an air conditioner "SAP-CE28B6" manufactured by SANYO Electric Co., and operated at intense cooling and the following experiment was conducted. That is, a test concerning a home air cleaner specified in the standards of Japanese Electronic Industry Association (JEMA) in JEM 1467 was repeated five times to measure the removal ratio for ammonia, acetoaldehyde and acetic acid, which were defined as 1-cycle. 4-cycle test also including the initial stage was repeated while repeating regeneration by the following method and the removable ratio on each cycle was calculated in accordance with the following equation (1). The results are shown in FIGS. 4 to 6 and Table 2. In JEM 1467, a load corresponding to five pieces of tobaccos is applied per a test. In this test, since the test is repeated for five times in one cycle, a load corresponding to 25 pieces of tobaccos is applied during 1 cycle. This is repeated for 4 cycles, so that the number of tobaccos for applying tobacco load is up to 100 in total.

[Calculation Method for Removal Ratio]

$$\text{Removal Ratio (\%)} = (1 - C/C_0) \times 100 \qquad (1)$$

C : initial gas concentration (ppm)
C$_0$ : residual gas concentration after 30 min (ppm)

[Regeneration Method]

After cleaning with water washing by using a neutral detergent, they were immersed in water for a whole day and night and then spontaneously dried at a room temperature. In this case, the deodorizing filter of Comparative Example 2 resulted in a problem that the activated carbon of the deodorant was partially peeled upon cleaning with water washing. Further, since activated carbon has no ion exchange function, the deodorizing function was not scarcely regenerated by the method. Accordingly, for the deodorizing filter of the comparative example, the gas removable ratio was measured by repeating the cycles without conducting regenerative operation to measure the gas removal ratio and the results are shown in Table 2 and Figure 4 to 6 as a comparison.

Table 2

| Gas as object | Ammonia | | Acetoaldehyde | | Acetic acid | |
|---|---|---|---|---|---|---|
| Specimen | Example | Comp · Example | Example | Comp · Example | Example | Comp. Example |
| Removal ratio upon 5 tobacco load (%) | 77 | 17 | 47 | 58 | 77 | 91 |
| Removal ratio upon 30 tobacco load (%) | 68 | 0 | 35 | 5 | 66 | 54 |
| Removal ratio upon 55 tobacco load (%) | 59 | - | 28 | - | 58 | 21 |
| Removal ratio upon 80 tobacco load (%) | 58 | - | 26 | - | 50 | 10 |

Then, based on the result obtained by the experiment described above, the number for duration and the number of day for duration were calculated according to the calculation method as defined in JEM 1467 according to the standard of Japan Electrical Manufacturer's Association (JEMA). The results are shown in Table 3. Each duration number in Table 3 is as shown below.

[Duration number for each gas ingredient ($K_1$ - $K_3$)]

The number of tobaccos when the removal ratio for each gas reaches 50% (when the ratio no more exceeds 50% even after regeneration).

[Number of total duration (Kt)]

Based on the duration number for each ingredient, the total duration number (Kt) was calculated in accordance with the following equation.

$$Kt = (K_1 + 2K_2 + K_3)/4$$

where, Kt : total duration number,
   $K_1$ : ammonia duration number,
   $K_2$ : acetoaldehyde duration number
   $K_3$ : acetic acid duration number

[Practical duration number]

Based on the total duration (Kt) described above, practical duration number (M) is determined according to the following equation:

$$M = 40 \times Kt$$

Table 3

| | Ammonia ($K_1$) | Acetoaldehyde ($K_2$) | Acetic acid ($K_3$) | total duration number (Kt) | Practical duration number (M) |
|---|---|---|---|---|---|
| Example | 100 | 0 | 80 | 45 | 1800 |
| Comparative Example | 0 | 11 | 31.5 | 13.4 | 536 |

As shown in Tables 2 and 3 and FIGS. 4 to 6 described above, it was confirmed that the deodorizing filter material according to the second invention can be regenerated for the deodorizing performance at a high level by the simple regeneration method described above and the durability can be improved greatly by regeneration.

## Claims

1. A deodorizing filter material in which a deodorant is deposited to three-dimensional fiber skeletons in which a pair of two-dimensional reticular skeletons are disposed in parallel and spaced apart by a predetermined distance from each other, and the pair of two-dimensional reticular skeletons are connected to each other by a number of connecting threads.

2. A deodorizing filter material as defined in claim 1, wherein the pair of two-dimensional reticular skeletons have substantially identical shape.

3. A deodorizing filter material as defined in claim 1 or 2, wherein the three-dimensional fiber skeleton is formed of polyester fibers, polyamide fibers, polypropylene fibers or composite fibers thereof.

4. A deodorizing filter material as defined in any one of claims 1 to 3, wherein the pair of two-dimensional reticular skeletons are formed by twisting two or more of fibers, and the connecting threads are formed by bridging a number of continuous threads between the two-dimensional reticular skeletons.

5. A deodorizing filter material as defined in any one of claims 1 to 4, wherein a binder layer is previously formed to the three-dimensional fiber skeleton, to which the deodorant is deposited.

6. A deodorizing filter material as defined in any one of claims 1 to 4, wherein a slurry formed by kneading a binder ingredient and a deodorant is impregnated to the three-dimensional fiber skeleton and dried thereby depositing the deodorant.

7. A deodorizing filter material as defined in any one of claims 1 to 4, wherein the slurry formed by kneading the binder ingredient and the deodorant is impregnated to the three-dimensional fiber skeleton and a deodorant is further deposited to the slurry and dried thereby depositing the deodorant.

8. A deodorizing filter material as defined in any one of claims 1 to 7, wherein an air permeable membrane is formed to one or all of the front surface, rear surface and the inside.

9. A deodorizing filter material as defined in any one of claims 1 to 8, wherein one or more of deodorants selected from coconut shell activated carbon, wooden activated carbon spherical petroleum pitch activated carbon, pellet molded activated carbon, natural zeolite, active white clay, surfactant, cationic or anionic-exchange resin, cationic or anionic-exchange fiber, chelate resin, chelate compound, inorganic cationic or anionic-adsorbent, inorganic synthetic chemical deodorant, a deodorant carrying, on a porous body a compound for chemically decomposing and deodorizing gas ingredients as an object by utilizing chemical reaction such as neutralization of dehydrating condensation, a deodorant carrying an oxidation or reduction catalyst comprising a noble metal or a base metal on a porous body, and a deodorant formed by carrying or coating a photo-excited catalyst such as titanium oxide on a porous body.

10. A deodorizing filter material in which one or more of different kinds of filter layers are laminated on one side or both sides of the deodorizing filter material as defined in any one of the claims 1 to 9.

**11.** A deodorizing filter material as defined in claim 10, wherein a deodorizing filter layer comprising a polyurethane foam having a three-dimensional reticular skeleton structure as a base is laminated as the different kind of the filter layer.

**12.** A deodorizing filter material as defined in claim 10 or 11, wherein a dust collecting filter layer having a dust collecting function is laminated as the different kind of the filter layer to provide a dust collecting function together with the deodorizing function.

**13.** A deodorizing filter material as defined in any one of claims 10 to 12, wherein the deodorizing filter material as defined in any one of claims 1 to 9 and different kind of the filter layer are laminated in a non-bonded state by using a clip-like or frame-like filter binding member.

**14.** A regenerative deodorizing filter material in which a deodorant having a gas adsorbing effect by an ion exchange function is deposited on the surface of the skeleton of the filter matrix having the three-dimensional structure.

**15.** A deodorizing filter material as defined in claim 14, wherein the filter matrix is a three-dimensional fiber skeleton in which a pair of two-dimensional reticular skeletons are disposed in parallel spaced apart by a predetermined distance from each other and the pair of two-dimensional reticular skeletons are connected by a number of connecting threads.

**16.** A deodorizing filter material as defined in claim 14 or 15, wherein the filter matrix comprises one or more synthetic fibers selected from polyester, polyamide and polypropylene.

**17.** A deodorizing filter material as defined in claim 14, wherein the filter matrix comprises polyurethane foams having three-dimensional reticular skeleton with no cell membranes.

**18.** A deodorizing filter material as defined in any one of claims 14 to 17, wherein the deodorant having the ion exchange function is an inorganic cationic-exchange deodorant selected from silicon dioxide series, zirconium series, titanium series, antimony series and tin series, or anionic-exchange deodorant selected from zinc oxide series, aluminum series and bismuth series.

**19.** A deodorizing filter material as defined in any one of claims 14 to 17, wherein the deodorant having the ion exchange function is an organic cationic or anionic-exchange deodorant selected from cationic or anionic-exchange resin, cationic or anionic ion exchange fibers, chelate resins and chelate compounds.

**20.** A deodorizing filter material as defined in any one of claims 14 to 17, wherein the deodorant having the ion exchange function is a composite deodorant comprising, in combination, one or more inorganic deodorants selected from the deodorants defined in claim 18 and one or more organic deodorants selected from deodorants described in claim 19.

**21.** A deodorizing filter material as defined in any one of claims 14 to 20, which can be regenerated by treating with water or an aqueous weakly basic or weakly acidic solution.

**22.** A deodorizing filter material which is regenerated from the deodorizing filter material as defined in any one of claims 14 to 21 with water or an aqueous weakly basic or weakly acidic solution.

**23.** A deodorizing filter material as defined claim 22, which is regenerated by treating with water or an aqueous weakly basic or weakly acidic solution while applying supersonic waves.

# FIG.1

# FIG.2

DIFFERENTIAL PRESSURE
GAUGE OF ORIFICE

SAMPLE

DIFFERENTIAL
PRESSURE GAUGE

BLOWER

# FIG.3

SAMPLE

STANDARD
GAS
GENERATOR

NITROGEN
CYLINDER

# FIG.4

**AMMONIA REMOVAL RATIO
ACCORDING TO JEMA**

# FIG.5

**ACETOALDEHYDE REMOVAL RATIO
ACCORDING TO JEMA**

# FIG.6

**ACETIC ACID REMOVAL RATIO
ACCORDING TO JEMA**

◆———◆ : EXAMPLE    △———△ : COMPARATIVE EXAMPLE

# EP 1 323 459 A1

| INTERNATIONAL SEARCH REPORT | International application No. |
|---|---|
| | PCT/JP01/05916 |

**A. CLASSIFICATION OF SUBJECT MATTER**
Int.Cl$^7$   B01D39/14, B01D39/16, A61L9/01, A61L9/16

According to International Patent Classification (IPC) or to both national classification and IPC

**B. FIELDS SEARCHED**

Minimum documentation searched (classification system followed by classification symbols)
Int.Cl$^7$   B01D39/14, B01D39/16, A61L9/01, A61L9/16

Documentation searched other than minimum documentation to the extent that such documents are included in the fields searched
Jitsuyo Shinan Koho          1926-1996      Toroku Jitsuyo Shinan Koho   1994-2001
Kokai Jitsuyo Shinan Koho   1971-2001      Jitsuyo Shinan Toroku Koho   1996-2001

Electronic data base consulted during the international search (name of data base and, where practicable, search terms used)
WPI (DIALOG)

**C. DOCUMENTS CONSIDERED TO BE RELEVANT**

| Category* | Citation of document, with indication, where appropriate, of the relevant passages | Relevant to claim No. |
|---|---|---|
| X | JP 5-7725 A (Matsushita Electric Ind. Co., Ltd.), 19 January, 1993 (19.01.93), Claims; Par. Nos. [0012] to [0017], [0029]; Figs. 1 to 3   (Family: none) | 1~16 |
| X | JP 9-57050 A (Bridgestone Corporation), 04 March, 1997 (04.03.97), Claims; Par. Nos. [0014] to [0034]   (Family: none) | 14,17~20 |
| A | JP 61-74610 A (Matsushita Electric Works, Ltd.), 16 April, 1986 (16.04.86), page 1, right column, line 9 to page 2, lower left column, line 12   (Family: none) | 1~23 |
| PX | JP 2001-79317 A (Sharp Corporation), 27 March, 2001 (27.03.01), Claims; Par. Nos. [0022] to [0039]   (Family: none) | 1~9,14~16, 18~23 |

☐ Further documents are listed in the continuation of Box C.     ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28 September, 2001 (28.09.01) | 09 October, 2001 (09.10.01) |

| Name and mailing address of the ISA/ | Authorized officer |
|---|---|
| Japanese Patent Office | |
| Facsimile No. | Telephone No. |

Form PCT/ISA/210 (second sheet) (July 1992)

17